# EUROPEAN PATENT APPLICATION

(11) **EP 2 128 137 A1**
(43) Date of publication of application: **02.12.2009**
(21) Application number: 07816770.7
(22) Date of filing: 07.11.2007
(51) Int. Cl.: C07D 221/22, A61K 31/439, A61P 25/16

(54) **SELECTIVE M4 RECEPTOR ANTAGONIST AND ITS MEDICAL USE**

(30) Priority: 30.12.2006 CN 200610171579
(71) Applicant: Institute of Pharmacology and Toxicology Academy of Military Medical Sciences P.L.A. China, Beijing 100850 (CN)
(72) Inventor: ZHONG, Bohua, Beijing 100850 (CN); ZHENG, Jianquan, Beijing 100850 (CN); WANG, Liyun, Beijing 100850 (CN); LIU, He, Beijing 100850 (CN); CHEN, Lanfu, Beijing 100850 (CN); LIU, Keliang, Beijing 100850 (CN)
(74) Representative: Fourcade, Emmanuelle
(86) International application number: PCT/CN2007/003158
(87) International publication number: WO 2008/080290

(57) **Abstract**

The present invention provides a selective M4 receptor antagonist, levorotatory demethylated phencynonate or its nontoxic pharmaceutically acceptable salt, a pharmaceutical composition comprising this compound, and a use thereof in the manufacture of a medicament for the treatment of motion dysfunction, such as, tremor, rigor and the like caused by Parkinson's disease (PD). The structure of the compound is shown in Formula IIa:

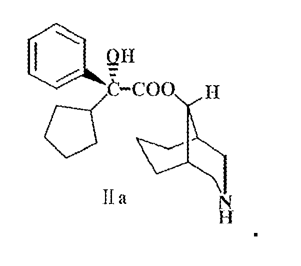

## Description

### Technical Filed

The present invention relates to a novel selective M4 receptor antagonist, levorotatory demethylated phencynonate or a nontoxic pharmaceutically acceptable salt thereof, to a pharmaceutical composition comprising the same compound as an active ingredient, and to the use of the compound in the manufacture of a medicament for the treatment of motion dysfunction, such as, tremor, rigor and the like caused by Parkinson's disease (PD).

### Background Art

M cholinoceptors are very important targets for medical treatment. The diseases including chronic obstructive pulmonary disease and urinary incontinence etc. caused by functional disorder of smooth muscle regulated by M1/M3 receptors have become high incidence of diseases worldwide. M2 receptor antagonists can promote the release of acetylcholine in brain and are useful for the treatment of Alzheimer's disease. M4 receptor can inhibit adenylate cyclase system, mainly exists in corpus striatum, and plays an important role in the onset and development of Parkinson's disease (PD). M4 receptor antagonists are effective for treatment of motion dysfunction such as tremor and rigor caused by PD. However, no selective M4 receptor antagonist has been found yet.

Hence, it is of very significant to find a selective M4 receptor antagonist for the development of new drugs with minor side effects for treatment of motion dysfunction such as tremor and rigor caused by PD, and for further studying to illustrate the physiological functions of M4 receptor.

Phencynonate hydrochloride, 2'-cyclopentyl-2'-phenyl-2'-hydroxyacetic acid 9α-[3-methyl-3-azabicyclo(3.3.1)-nonanyl] ester hydrochloride, as shown in Formula (I), is a selective anticholine drug and has been used clinically in the prevention and treatment of motion sickness such as car- and sea-sickness. Chinese patent application publication No. CN1089838 A and US patent No. 6,028,198 disclose the use of phencynonate hydrochloride as a drug for the treatment of motion sickness such as car-, sea- and air-sickness. Chinese patent No. 97125424.9, British patent No. GB2,297,255 and Spanish patent No. ES549796 disclose processes for preparing phencynonate hydrochloride. Chinese patent application No. 01104881.6 claims the use of phencynonate hydrochloride for the treatment of Parkinson's disease/syndrome. Chinese patent application No. 01104881.6 claims the medical use of phencynonate hydrochloride for the treatment or remission of the acute episodes of vertigoes such as Meniere's disease and positional vertigo.

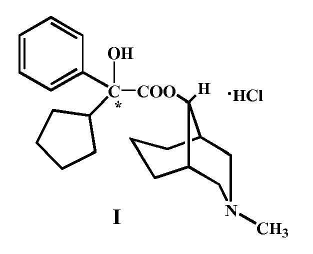

Formula (I) shows that the molecular structure of phencynonate hydrochloride contains a chiral carbon atom, and thus has a pair of optical isomers. Currently, the racemic form of phencynonate hydrochloride is used in clinic.

Without any external chiral influences, both optical isomers of a chiral molecule have the same physical or chemical properties, such as melting point, solubility, chromatography retention time, IR spectrum and nuclear magnetic resonance spectrum. However, different optical isomers exhibit opposite optical activities, i.e., they can rotate a plane polarized light clockwise (dextrorotation) or anti-clockwise (levorotation).

Prefixes D and L or R and S can be used to represent the absolute configurations of chiral centers of optical isomers. Prefixes d and I or (+) and (-) can be used to represent the optical rotation properties of molecules. For example, d-tartaric acid or (+)-tartaric acid indicates that this isomer is a dextrorotary isomer, while I-tartaric acid or (-)-tartaric acid indicates that the isomer is a levorotary isomer.

### Contents of the Invention

One object of the present invention is to provide a selective M4 receptor antagonist, levorotatory demethylated phencynonate or a nontoxic pharmaceutically acceptable salt thereof. Another object of the present invention is to provide a use of levorotatory demethylated phencynonate or a nontoxic pharmaceutically acceptable salt thereof as a selective M4 receptor antagonist for the treatment of motion dysfunction, such as, tremor, rigor and the like caused by Parkinson's disease (PD).

It has been surprisingly found that levorotatory demethylated phencynonate as shown in Formula IIa exhibits selective antagonistic effect on M4 receptor. In a competitive binding assay of using [³H]NMS as ligand, levorotatory demethylated phencynonate exhibits a competitive antagonistic effect on M4 receptor (IC₅₀=4.4×10⁻³nmmol/L), which is 145 times greater than that on M1 receptor (IC₅₀= 0.64 nM), 125 times greater than that on M2 receptor, 9 times greater than that on M3 receptor (IC₅₀ = 4×10⁻² nM), and 7 times greater than that on M5 receptor (IC₅₀ = 3×10⁻² nM). Thus, levorotatory demethylated phencynonate is a selective M4 receptor antagonist.

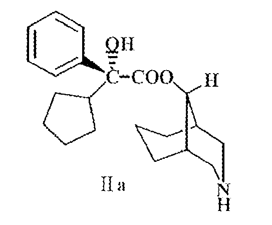

The term "pharmaceutically acceptable salt" used herein refers to a pharmaceutically acceptable inorganic or organic salt. The compound of formula IIa with a basic group according to the present invention can react with a suitable inorganic acid to form a pharmaceutically acceptable salt, such as sulfate, hydrochloride, hydrobromide, phosphate; or react with a suitable organic acid to form a pharmaceutically acceptable salt, such as acetate, oxalate, citrate, gluconate, succinate, tartrate, p-tosylate, mesylate, benzoate, lactate, maleate, etc.

The compound of the present invention or a pharmaceutically acceptable salt thereof can form a solvate, such as hydrate, alcoholate, etc. The compound may also be a prodrug or in a form that can release the active ingredient upon metabolized in vivo. The techniques for selecting and preparing suitable prodrug derivatives are known to a person skilled in the art.

As abovementioned, the compound of Formula IIa of the present invention can be used for the treatment of motion dysfunction, such as, tremor, rigor and the like caused by Parkinson's disease (PD).

The compound of the present invention or a pharmaceutically acceptable salt thereof can be administered individually or in a form of pharmaceutical composition. The pharmaceutical composition of the present invention can be formulated into various suitable dosage forms depending on the routes of administration. One or more physiologically acceptable carriers including excipients and auxiliary agents are used for facilitating the processing of the active compound into pharmaceutically acceptable dosage forms. Suitable dosage forms depend on the selected routes of administration, and can be manufactured in accordance with the common knowledge in the art.

The compound of the present invention can be administrated orally, parentally or topically, preferably orally and by injection. Dosage forms for oral administration include capsules, tablets, etc. For patients with dysphagia, sublingual tablets or other non-swallowed preparations may be suitable dosage forms for administration. The compound of the present invention may also be formulated for parental administration or transdermal administration or transmucosal administration, or formulated into a suppository or an implant. Those skilled in the art would understand that a suitable drug delivery system (DDS) can be used for the compound of the present invention to achieve better effects.

In addition, it is noted that the used dosage and modes of the compound of the present invention depend on many factors including age, weight, gender, natural health condition and nutritional state of patient, activity strength of the compound, duration of administration, rate of metabolism, severity of a condition, and subjective judgment of the attending physician. Preferable dosage for use is in the range of 0.01-100 mg/kg of body/day.

The compound of Formula IIa can be prepared by a synthetic method comprising: removing N-methyl group of phencynonate using 2,2,2-trichloroethoxyformic acid chloride so as to obtain demethylated phencynonate (III), reacting it with single enantiomer of N-p-tosyl-glutamic acid to form a salt, recrystallizing the salt for several times to obtain an optically pure salt of demethylated phencynonate optical isomer with N-p-tosyl-glutamic acid optical isomer [II(-)·IV(+) and II(+)·IV(-)], basifying the salt to obtain an optically pure single isomer of N-demethylate phencynonate (levorotatory demethylated phencynonate IIa and dextrorotatory demethylated phencynonate IIb). The scheme of the synthetic method is shown as follows:

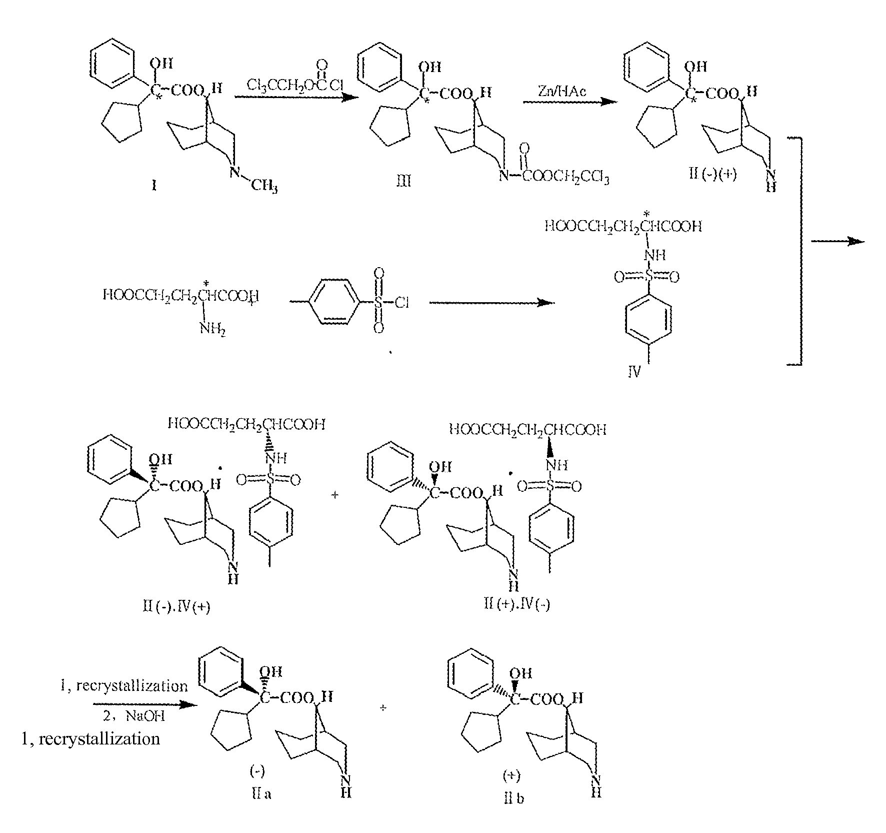

### Concrete Modes for Carrying Out the Invention

The following examples are used to specifically illustrate the present invention, but are not intended to restrict the present invention in any way.

### Example 1: Synthesis of 2'-cyclopentyl-2'-phenyl-2'-hydroxyacetic acid-9α-[N(2,2,2-trichloroethoxyformyl)-3-aza-bicyclo[3.3.1]nonanyl]ester (III)

10g (25mmol) of 2'-cyclopentyl-2'-phenyl-2'-hydroxyacetic acid -9α-[3-methyl-3-azabicyclo(3.3.1)nonanyl]ester hydrochloride (I) was added to 80mL ethyl ether, basified by adding aqueous ammonia to convert (I) into a free base dissolved in ethyl ether, then washed with water, dried and vaporized under a reduced pressure to remove ethyl ether. 20mL anhydrous benzene was added to the residue and then vaporized under a reduced pressure to dryness, and this step was repeated for three times. Finally, the obtained free base was dissolved in 30mL anhydrous benzene to obtain a solution, 8.3g 2,2,2-trichloroethoxyformic acid chloride in 20mL benzene was added to the solution and then 300mg anhydrous potassium carbonate was added. The solution was heated under stirring in an oil bath at 85°C for 5 hours, cooled and filtrated to remove solids. The filtrate was vaporized under a reduced pressure to dryness, the obtained residue was dissolved in 40mL ethyl ether, washed with diluted aqueous ammonia and water sequentially, dried over anhydrous potassium carbonate, vaporized under a reduced pressure to remove solvent therein to obtain 12.2g of a yellowish transparent viscose (III) in a yield of 94%. Element analysis: theoretical values (%) for C₂₄H₃₀NO₅Cl₃: C 55.56, H 5.83, N 2.70; experimental values (%): C 54.62, H 5.86, N 2.73. Mass spectrum (FAB) m/z (%): 518 (M⁺-1, 14.67 ), 317(18.00), 175(100.00).

### Example 2: Synthesis of 2'-cyclopentyl-2'-phenyl-2'-hydroxyacetic acid-9-[3-aza-bicyclo(3.3.1)nonanyl]ester (II)

11g (21mmol) of (III) was dissolved in 30mL ethyl acetate, and 25mL 90% acetic acid solution was added. 7g of zinc powder was added in several portions with stirring, and the temperature was kept at 50°C. After all zinc powder was added, the obtained mixture was heated under stirring in a water bath at 40°C for 2 hours. Then, 25mL 90% acetic acid solution and 5g of zinc powder were added, and heated under stirring in a water bath at 50°C for 12 hours. The reaction is complete detected by a silica gel thin layer chromatography. Solids were filtrated off, and washed with ethanol. Filtrates were combined and dried by vaporization under a reduced pressue, then 150mL ethyl ether was added, basified using 5% NaOH solution and dissolve free base in ethyl ether. Ether layer was separated, washed with water till neutral, dried over anhydrous magnesium sulfate, and vaporized under a reduced pressure to remove solvent to obtain a yellowish solid. The solid was recrystallized from methanol to obtain a colorless crystal (II) of 5.3g in a yield of 73%. Melting point: 124-126°C. Element analysis: theoretical values (%) for C₂₁H₂₉NO₃: C 73.44, H 8.51, N 4.08; experimental values (%): C 73.49, H 8.66, N 3.96. ¹H-NMR: δ (ppm, CD₃Cl), 7.70(m, 2H), 7.31 (m, 3H), 4.63(s, 1H), 3.86(s, 1H), 2.96-3.19(m, 5H), 2.16(m, 1H), 2.00(m, 1H), 1.90(s, 1H), 1.32-1.69(m, 14H).

### Example 3: Preparation of (-)-2'-cyclopentyl-2'-phenyl-2'-hydroxyacetic acid-9-[3-aza-bicyclo(3.3.1)nonanyl]ester (levorotatory demethylated phencynonate, IIa)

14.7g (0.1mol) L-glutamic acid was dissolved in 100mL 2N NaOH, heated to about 70°C in a water bath, then 22.8g (0.12mol) p-tosylchloride was added in portions under stirring over about half an hour. NaOH solution was added dropwisely continuously to keep pH at ≥9 and the reaction was performed at 70°C under stirring for 1 hour. After being cooled to room temperature, the reaction is cooled in an ice-salt bath to below 0°C, a concentrated hydrochloric acid was added dropwisely until pH was about 3, and extracted with 300mL ethyl acetate in three portions. All extracts were combined, heated under reflux, decolored with active carbon, filtrated, dried over anhydrous calcium chloride as desiccant, filtrated again, and the desiccant was washed with anhydrous ethyl acetate. The obtained solution was concentrated to 80mL, and cooled in an ice-salt bath to precipitate crystalline L-(+)-N-p-tosylglutamic acid of 27.7g in a yield of 92%. [α]_{D}²⁰ = +22.6 (room temperature, ethyl acetate, c = 1.10), melting point: 130-132°C.

6.8g (0.02mol) of racemic 2'-cyclopentyl-2'-phenyl-2'-hydroxyacetic acid-9-[3-aza-bicyclo(3.3.1)nonanyl]ester was dissolved in 200mL 50°C anhydrous ethanol, then 6.0g (0.02mol) (+)-N-p-tosylglutamic acid in 50mL anhydrous ethanol solution was added dropwisely. After complete of addition in dropwise, the reaction liquid was cooled to room temperature and stood overnight. (-)-2'-cyclopentyl-2'-phenyl-2'-hydroxyacetic acid-9-[3-aza-bicyclo(3.3.1)nonanyl]ester (+) N-p-tosylglutamic acid salt (-) II • (+) IV was collected by filtration. The obtained salt was recrystallized from ethanol (10mL/g), until the optical rotation value becomes stable.

The obtained salt was placed in 180mL anhydrous ethyl ether, and basified by adding 5% KOH solution. Ethyl ether layer was separated, washed with water, and dried to recover ethyl ether to obtain (-)-2'-cyclopentyl-2'-phenyl-2'-hydroxyacetic acid-9-[3-aza-bicyclo(3.3.1)nonanyl]ester (IIa) of 1.7g in a yield of 50%. [α]_{D}²⁰ = -13.8 (room temperature, ethanol, c = 0.4), melting point: 138-139°.

### Example 4: Competitive binding assay of the compound for various subtypes of M receptors

CHO cells stably expressing M1-5 receptor subtypes were used to prepare membrane proteins, and the competitive binding assay was conducted by using [³H]NMS as ligand. Affinities of IIa to different M receptor subtypes were compared, and the results were shown in Table 1.

**Table 1: Competitive binding assay results of IIa to different M receptor subtypes [IC₅₀, (M)]**

| Compound | M1 | M2 | M3 | M4 | M5 |
|---|---|---|---|---|---|
| IIa | 6.4×10⁻⁷ | 5.5×10⁻⁷ | 3.9×10⁻⁸ | 4.4×10⁻⁹ | 5.0×10⁻⁸ |

The assay results showed that levorotatory demethylated phencynonate exhibits a relative selectivity to M4 receptor subtype, and its antagonistic effect on M4 receptor was 145 times greater than that on M1 receptor, 125 times greater than that on M2 receptor, 9 times greater than that on M3 receptor, and 7 times greater than that on M5 receptor.

### Example 5: Acute toxicity test in mice

Kunming species of mice with a body weight of 18-22g, half male and half female, were randomly grouped, and administrated by intraperitoneal injection. The response of animals was observed and the number of dead animals was counted over 24 hours after administration. LD₅₀ was calculated to be 490mg/Kg using Bliss method.

## Claims

1. Levorotatory demethylated phencynonate represented by Formula IIa, 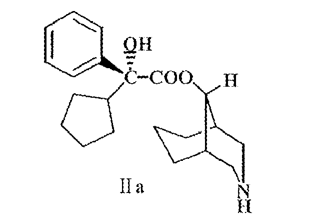 or a nontoxic pharmaceutically acceptable salt thereof.

2. The nontoxic pharmaceutically acceptable salt of levorotatory demethylated phencynonate according to claim 1, comprising a pharmaceutically acceptable salt formed with an inorganic acid, such as sulfate, hydrochloride, hydrobromide and phosphate; or a pharmaceutically acceptable salt formed with an organic acid, such as acetate, oxalate, citrate, gluconate, succinate, tartrate, p-tosylate, mesylate, benzoate, lactate, and maleate.

3. Levorotatory demethylated phencynonate or a nontoxic pharmaceutically acceptable salt thereof according to claim 1 or 2, which may form a solvate, such as hydrate, alcoholate.

4. Levorotatory demethylated phencynonate or a nontoxic pharmaceutically acceptable salt thereof according to claim 1 or 2, wherein the levorotatory demethylated phencynonate or a nontoxic pharmaceutically acceptable salt thereof can be a prodrug or in a form that can release the active ingredient upon metabolized in vivo.

5. A pharmaceutical composition comprising the levorotatory demethylated phencynonate or a nontoxic pharmaceutically acceptable salt thereof according to any one of claims 1 to 4 as the active ingredient, and suitable excipients.

6. Use of the levorotatory demethylated phencynonate or a nontoxic pharmaceutically acceptable salt thereof according to any one of claims 1 to 4 in the manufacture of a medicament for the treatment of motion dysfunction, such as tremor, rigor and the like caused by Parkinson's disease (PD).
